# EUROPEAN PATENT SPECIFICATION

(11) **EP 1 674 461 B1**
(45) Date of publication and mention of the grant of the patent: **15.04.2009**
(21) Application number: 04792402.2
(22) Date of filing: 14.10.2004
(51) Int. Cl.: C07D 317/22, C07D 317/16, C07D 317/20, C07D 317/24, C07D 317/26, C08F 24/00

(54) **FLUORINE-CONTAINING DIOXOLANE COMPOUNDS AND FLUORINE-CONTAINING POLYMERS**
FLUORHALTIGE DIOXOLANVERBINDUNGEN UND FLUORHALTIGE POLYMERE
COMPOSES DIOXOLANES FLUORES ET POLYMERES FLUORES

(30) Priority: 16.10.2003 JP 2003356939
(43) Date of publication of application: 28.06.2006
(73) Proprietor: ASAHI GLASS COMPANY LTD., Tokyo 100-8405 (JP)
(72) Inventor: SUGIYAMA, Norihide, Asahi Glass Company, Limited, Yokohama-shi, Kanagawa 2218755 (JP); ITO, Masahiro, Asahi Glass Company, Limited, Yokohama-shi, Kanagawa 2218755 (JP); MUROTANI, Eisuke, Asahi Glass Company, Limited, Yokohama-shi, Kanagawa 2218755 (JP); OKAZOE, Takashi, Asahi Glass Company, Limited, Yokohama-shi, Kanagawa 2218755 (JP)
(74) Representative: Müller-Boré & Partner Patentanwälte
(86) International application number: PCT/JP2004/015171
(87) International publication number: WO 2005/037818

(56) References cited:
- WO-A1-02/10106
- WO-A1-02/46811
- WO-A1-03/037885
- JP-A- 5 339 255
- JP-A- 60 104 092
- US-A- 3 308 107
- US-A- 3 475 456
- US-A- 5 408 020

## Description

### TECHNICAL FIELD

The present invention relates to a fluorinated dioxolan compound which can be used as a raw material for a fluorinated polymer excellent in characteristics such as heat resistance, mechanical strength, transparency and rigidity, and a novel fluorinated polymer.

### BACKGROUND ART

US Patent 3,307,330 (hereinafter referred to as Patent Document 1) and US Patent 3,308,107 (hereinafter referred to as Patent Document 2) disclose perfluoro (2-methylene-4-methyl-1,3-dioxolan) represented by the following formula (15) as a compound having a perfluoro (2-methylene-1,3-dioxolan) skeleton:

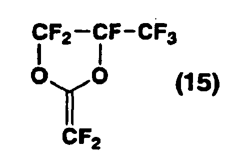

Further, Patent Documents 1 and 2 disclose that a polymer having the compound (15) polymerized, is soluble in e.g. a fluorinated solvent and may be used for an adhesive or a coating material, and that the polymer may be formed into a film, which can be used as a gas permeable film material.

JP-A-5-339255 (hereinafter referred to as Patent Document 3) discloses a compound represented by the following formula (16) (wherein R² is a C₂₋₇ polyfluoroalkyl group):

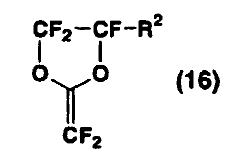

Further, Patent Document 3 discloses that a fluorinated polymer soluble in a special solvent, can be obtained by homopolymerization of the compound (16) or by copolymerization thereof with another comonomer, and that the polymer can be applied to e.g. a low reflection coating or a thin film.

Patent Documents 1 to 3 disclose a compound having one perfluoro-(2-methylene-1,3-dioxolan) skeleton in the same molecule, but do not disclose a compound having two or more such skeletons.

The above compound (15) is prepared from a fluoropyruvic acid fluoride and hexafluoropropylene oxide, which are expensive and difficult to handle. Whereas, the compound (16) is prepared from hexafluoropropylene oxide and α-ketocarboxylic acid fluoride obtained by oxidation and epoxidation of a perfluoroolefin, but this process is very complex.

Thus, the compounds (15) and (16) were not suitable as raw materials to be used for industrial production of a fluorinated polymer useful as a highly functional fluorinated material.

### DISCLOSURE OF THE INVENTION

### OBJECTS TO BE ACCOMPLISHED BY THE PRESENT INVENTION

An object of the present invention is to provide a novel fluorinated dioxolan compound by an industrially advantageous method, whereby a fluorinated polymer excellent in characteristics such as heat resistance, mechanical strength, transparency and rigidity, can be synthesized. Further, another object of the present invention is to provide a novel compound useful as an intermediate for the production of the novel fluorinated dioxolan compound. Further, still another object of the present invention is to provide a novel polymer excellent in characteristics such as heat resistance, mechanical strength, transparency and rigidity.

The present invention is as follows:
(1) A compound represented by the following formula (1A) (wherein Q^{f1} represents a single bond, an oxygen atom, a C₁₋₅ perfluoroalkylene group, or a C₁₋₅ perfluoroalkylene group having an etheric oxygen atom inserted between carbon-carbon atoms): 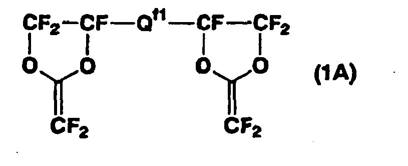
(2) A compound represented by the following formula (1B) (wherein Q^{f1} represents a single bond, an oxygen atom, a C₁₋₅ perfluoroalkylene group, or a C₁₋₅ perfluoroalkylene group having an etheric oxygen atom inserted between carbon-carbon atoms, and X² is a fluorine atom, or a group represented by -OR (wherein R is a hydrogen atom, a C₁₋₅ alkyl group, or a C₁₋₅ alkyl group having an etheric oxygen atom inserted between carbon-carbon atoms)): 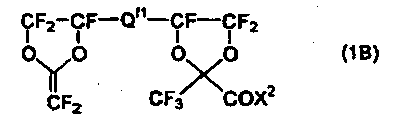
(3) A compound represented by the following formula (5) (wherein Q¹ represents a single bond, an oxygen atom, a C₁₋₅ alkylene group, or a C₁₋₅ alkylene group having an etheric oxygen atom inserted between carbon-carbon atoms): 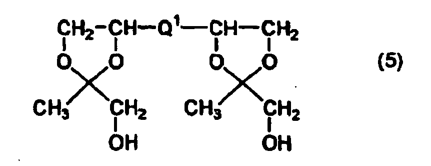
(4) A compound represented by the following formula (4) (wherein Q¹ represents a single bond, an oxygen atom, a C₁₋₅ alkylene group, or a C₁₋₅ alkylene group having an etheric oxygen atom inserted between carbon-carbon atoms, and two R^{f2} in the formula may be the same or different, and each represents a C₁₋₁₀ perfluoroalkyl group, or a C₁₋₁₀ perfluoroalkyl group having an etheric oxygen atom inserted between carbon-carbon atoms): 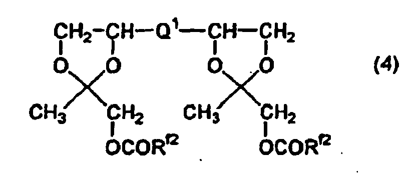
(5) A compound represented by the following formula (3) (wherein Q^{f1} represents a single bond, an oxygen atom, a C₁₋₅ perfluoroalkylene group, or a C₁₋₅ perfluoroalkylene group having an etheric oxygen atom inserted between carbon-carbon atoms, and two R^{f2} in the formula may be the same or different, and each represents a C₁₋₁₀ perfluoroalkyl group, or a C₁₋₁₀ perfluoroalkyl group having an etheric oxygen atom inserted between carbon-carbon atoms): 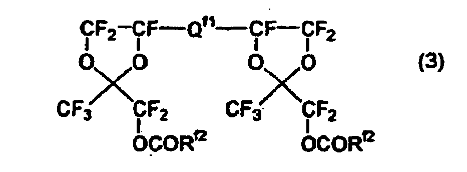
(6) A compound represented by the following formula (2) (wherein Q^{f1} represents a single bond, an oxygen atom, a C₁₋₅ perfluoroalkylene group, or a C₁₋₅ perfluoroalkylene group having an etheric oxygen atom inserted between carbon-carbon atoms, and two X² in the formula may be the same or different, and each represents a fluorine atom, or a group represented by -OR (wherein R represents a hydrogen atom, a C₁₋₅ alkyl group, or a C₁₋₅ alkyl group having an etheric oxygen atom inserted between carbon-carbon atoms)): 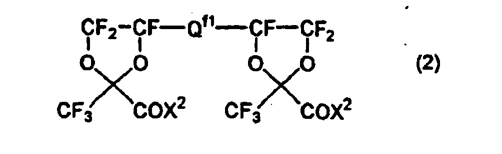
(7) A polymer essentially comprising at least one type of repeating units selected from a repeating unit represented by the following formula (9), a repeating unit represented by the following formula (10) and a repeating unit represented by the following formula (11) (wherein Q^{f1} represents a single bond, an oxygen atom, a C₁₋₅ perfluoroalkylene group, or a C₁₋₅ perfluoroalkylene group having an etheric oxygen atom inserted between carbon-carbon atoms, and, in a case where the polymer essentially comprises at least two types of repeating units, Q^{f1} in the formulae may be the same or different, X² represents a fluorine atom, or a group represented by -OR (wherein R represents a hydrogen atom, a C₁₋₅ alkyl group, or a C₁₋₅ alkyl group having an etheric oxygen atom inserted between carbon-carbon atoms)): 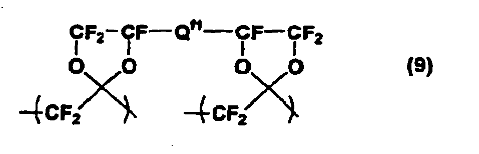 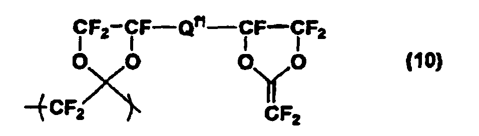 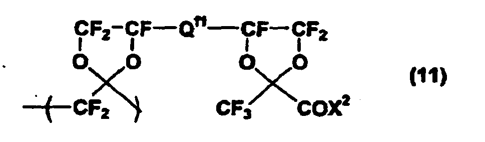
(8) A method for producing a polymer, characterized by polymerizing a compound represented by the following formula (1A) and/or a compound represented by the following formula (1B), or polymerizing a compound represented by the following formula (1A) and/or a compound represented by the following formula (1B), and another polymerizable compound (wherein Q^{f1} represents a single bond, an oxygen atom, a C₁₋₅ perfluoroalkylene group or a C₁₋₅ perfluoroalkylene group having an etheric oxygen atom inserted between carbon-carbon atoms, and in a case where the polymer essentially comprises at least two types of repeating units Q^{f1} in the formulae may be the same or different, X² represents a fluorine atom or a group represented by -OR (wherein R represents a hydrogen atom, a C₁₋₅ alkyl group, or a C₁₋₅ alkyl group having an etheric oxygen atom inserted between carbon-carbon atoms)):

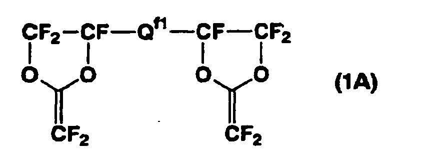

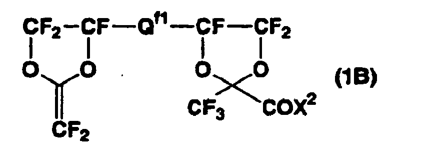

### BEST MODE FOR CARRYING OUT THE INVENTION

In this specification, "a compound represented by the formula (1A)" will be represented also as "the compound (1A)". Compounds and repeating units represented by other formulae will also be represented in the same manner.

The compounds in the present invention and general outlines of the processes for the production of the compounds, may be represented by the following preparation routes.

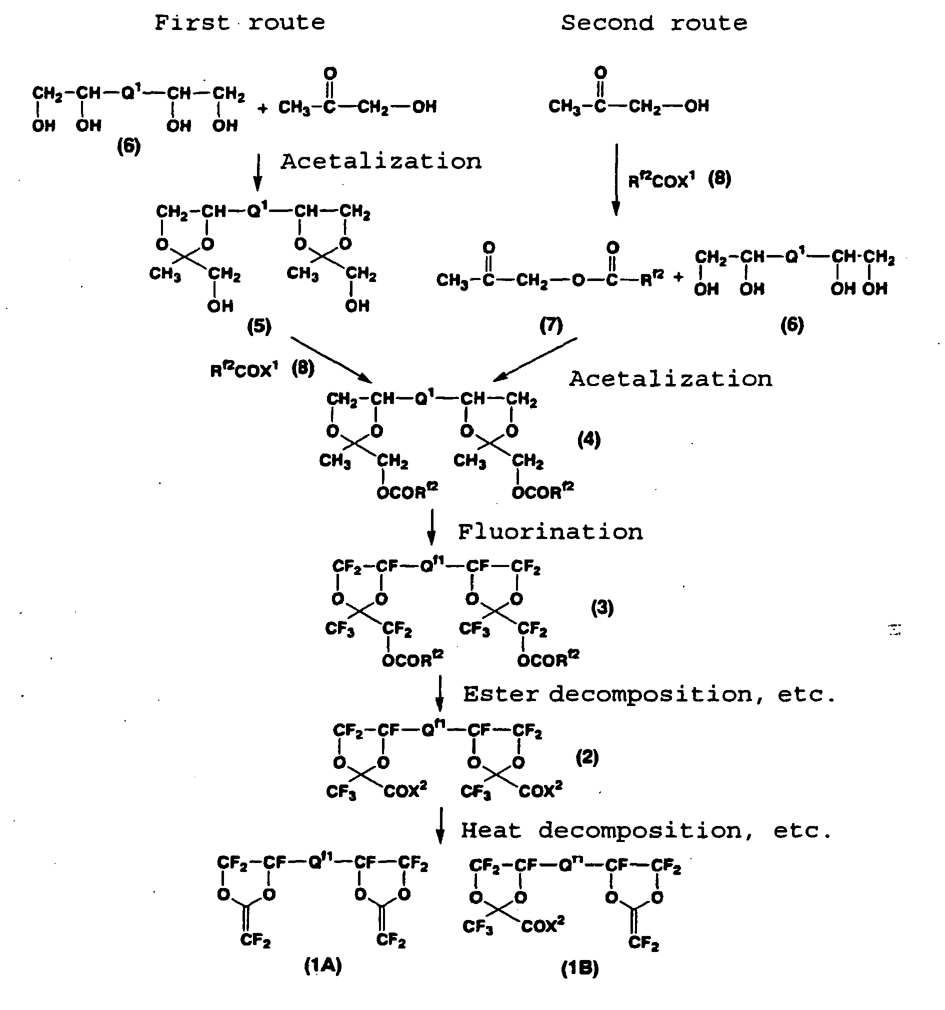

The compound (1A) and the compound (1B) are novel compounds, and they can be polymerized to form novel fluorinated polymers excellent in characteristics such as heat resistance, mechanical strength, transparency and rigidity. Further, the compound (1A) has two polymerizable groups in its molecule and thus is useful also as a crosslinking agent for e.g. a fluorinated polymer. Further, the compounds (2) to (5) are novel compounds useful as intermediates for the production of the compound (1A) and the compound (1B).

In each formula for the present invention, Q¹ represents a single bond, an oxygen atom, a C₁₋₅ alkylene group, or a C₁₋₅ alkylene group having an etheric oxygen atom inserted between carbon-carbon atoms. In a case where Q¹ is a C₁₋₅ alkylene group, or a C₁₋₅ alkylene group having an etheric oxygen atom inserted between carbon-carbon atoms, Q¹ may have a straight chain structure or a branched structure.

Q^{f1} is a group corresponding to Q¹ and represents a single bond, an oxygen atom, a C₁₋₅ perfluoroalkylene group, or a C₁₋₅ perfluoroalkylene group having an etheric oxygen atom inserted between carbon-carbon atoms. Q^{f1} is preferably a group such as -CF₂-, -(CF₂)₂-, -(CF₂)₃-, -CF₂OCF₂-, -(CF₂)₂O(CF₂)₂-, or -(CF₂)₃O(CF₂)₃-.

In a case where Q¹ and Q^{f1} are single bonds, two carbon atoms to which Q¹ or Q^{f1} is bonded, are directly bonded to each other. Further, the absolute configuration of an asymmetric carbon atom in the above chemical formulae is not particularly limited and may be R or S, and in the after-mentioned production method, the absolute configuration will not be maintained in a usual case.

Further, the structure of Q¹ or Q^{f1} may be a symmetric structure or an asymmetric structure, preferably a symmetric structure. In a case where the structure of Q^{f1} is an asymmetric structure, the compound (1B) will usually be a mixture of two compounds. In this specification, the direction of Q^{f1} in the formula (1B) is not particularly limited, and one or both of the decomposition reaction products will be generally shown.

X¹ represents a fluorine atom or a chlorine atom. X² represents a fluorine atom or a group represented by -OR (wherein R is a hydrogen atom, a C₁₋₅ alkyl group, or a C₁₋₅ alkyl group having an etheric oxygen atom inserted between carbon-carbon atoms).

R^{f2} may be the same or different and each represents a C₁₋₁₀ perfluoroalkyl group, or a C₁₋₁₀ perfluoroalkyl group having an etheric oxygen atom inserted between carbon-carbon atoms. As the C₁₋₁₀ perfluoroalkyl group, a C₁₋₅ perfluoroalkyl group is preferred. As the C₁₋₁₀ perfluoroalkyl group having an etheric oxygen atom inserted between carbon-carbon atoms, a C₁₋₅ perfluoroalkyl group having an etheric oxygen atom inserted between carbon-carbon atoms is preferred.

With reference to the above preparation routes, the present invention will be described.

The production of the compound (4) is preferably carried out by the first route or the second route.

The first route is a route wherein a tetraol compound of the formula (6) and hydroxyacetone are subjected to an acetalization reaction to obtain a compound (5), and then, the compound (5) and a fluorinated acyl halide compound of the formula (8) are subjected to a condensation reaction to obtain the compound (4).

The compound (6) has a skeleton having two hydroxyl groups bonded to the adjacent carbon atoms (an ethyleneglycol skeleton) at each terminal of the molecule. The compound (6) can be obtained by oxidation and hydrolysis of an alkadiene compound or a compound having an etheric oxygen atom inserted between carbon-carbon bonds of an alkylene group moiety of an alkadiene compound. The alkadiene compound may, for example, be butadiene, 1,4-pentadiene, 1,5-hexadiene, 1,7-octadiene, divinyl ether or diallyl ether.

The acetalization reaction in the first route is preferably carried out in the presence of an acid catalyst and an orthoformic acid ester, or an acid catalyst and an orthoacetic acid ester. The acid catalyst is preferably a Lewis acid, and for example, an inorganic acid such as hydrochloric acid or sulfuric acid, an organic acid such as p-toluenesulfonic acid, or a solid acid such as an acidic ion exchange resin, may be mentioned. In the acetalizaion reaction, the lower limit of the reaction temperature is preferably 0°C, and the upper limit is preferably the lowest boiling point among the boiling points of the compounds to be used in the reaction. In the acetalization reaction, a formic acid ester or an acetic acid ester may sometimes be formed as a by-product. To remove such a by-product, it is preferred to bring the temperature to a level higher than the boiling point of the by-product at the end of the reaction thereby to gasify the by-product and discharge it out of the reaction system.

Then, the compound (5) and the compound (8) are subjected to a condensation reaction to obtain the compound (4).

The reaction temperature for the condensation reaction is preferably from -50°C to 100°C. Further, in a case where X¹ in the formula (8) is a fluorine atom, HF may sometimes be formed as a by-product. To remove such a by-product, a HF-capturing agent such as NaF or KF may preferably be added to the reaction system. Or, it is preferred to let an inert gas flow in the reaction system so that HF will be accompanied therewith and discharged out of the reaction system. Further, when X¹ is a chlorine atom, HCl will be formed, and therefore, it is preferred to add an acid-binding agent.

The second route is a route wherein hydroxyacetone and the compound (8) are subjected to a condensation reaction to obtain a compound (7), and then, the compound (7) and the compound (6) are subjected to an acetalization reaction to obtain the compound (4).

The condensation reaction and the acetalization reaction in the second route can be carried out in the same manner and conditions as the condensation reaction and the acetalization reaction in the first route.

The second route is a method wherein in the purification of the compound (4), a non-reacted product such as the compound (7) can readily be removed. Accordingly, the production of the compound (4) is preferably carried out by the second route.

Then, the compound (4) is reacted with fluorine (F₂) in a liquid phase for perfluorination to obtain a compound (3) .

When reacted with fluorine in a liquid phase, the compound (4) will be fluorinated. The fluorination is a reaction wherein hydrogen atoms in the compound (4) are substituted by fluorine atoms. In the present invention, the fluorination reaction is carried out until all of hydrogen atoms in the compound (4) will be substituted by fluorine atoms (i.e. until perfluorinated).

The fluorination reaction in the liquid phase (hereinafter referred to as the liquid phase fluorination reaction) is preferably carried out in a solvent in accordance with a prescribed method. The solvent is preferably one capable of dissolving both the compound (4) and the compound (3) as a product of the liquid phase fluorination reaction. As such a solvent, a fluorinated solvent inert to the liquid phase fluorination reaction is preferred, and a solvent capable of dissolving at least 1 mass% of the compound (3) is more preferred, and a solvent capable of dissolving at least 5 mass% of the compound (3) is particularly preferred. Specifically, a perfluoroalkane (such as FC-72, trade name, manufactured by 3M), a perfluoroether (such as FC-75 or FC-77, trade name, manufactured by 3M) a perfluoropolyether (KRYTOX, trade name, manufactured by DuPont FOMBLIN or GALDEN, trade name, manufactured by Ausimont, or DEMNAM, trade name, manufactured by Daikin Industries, Ltd.), a chlorofluorocarbon, or a perfluoroalkylamine (such as FC-43, trade name, manufactured by 3M) may, for example, be mentioned. Further, the compound (3) or the compound (4) may itself be used as a solvent.

From the viewpoint of the selectivity, the amount of fluorine to be used for the fluorination reaction is preferably maintained so that the amount of fluorine to the amount of hydrogen atoms contained in the compound (4) will always be in an excessive equivalent from the beginning to the end of the reaction. And it is particularly preferred to maintain the amount of fluorine to the amount of hydrogen atoms to be at least 1.05 times by mol.

Further, as the fluorine, 100% fluorine gas may be used as it is, or a mixed gas having such a fluorine gas diluted with an inert gas, may be employed. As an inert gas, nitrogen gas or argon gas may, for example, be employed. The concentration of fluorine in the mixed gas is preferably at least 10 vol%, more preferably at least 20 vol%.

The reaction temperature for the fluorination reaction is usually preferably from -60°C to the boiling point of the compound (4), and from the viewpoint of the reaction yield, selectivity and industrial operation efficiency, it is more preferably from -50°C to +100°C, further preferably from -20°C to +50°C. The reaction pressure is not particularly limited, and from the viewpoint of the reaction yield, selectivity and industrial operation efficiency, it is particularly preferably from normal pressure to 2 MPa (gage pressure, the same applies hereinafter).

Then, from the compound (3), a compound (2) will be produced. As such a method, the following method 1 or 2 is preferred.
Method 1: A method wherein by a decomposition reaction of the ester bond in the compound (3), the -CF₂OCOR^{f2} group in the compound (3) is converted to a -COF group to obtain a compound of the formula (2) wherein X² is a fluorine atom (hereinafter referred to as a compound (2-1)).
Method 2: A method wherein the compound (3) is reacted with a compound represented by the formula R-OH (wherein R is as defined above and is preferably an alkyl group or a hydrogen atom, particularly preferably a C₁₋₄ alkyl group or a hydrogen atom) to obtain a compound of the formula (2) wherein X² is -OR (hereinafter referred to as a compound (2-2)).

These reactions may generally be represented by the following formulae.

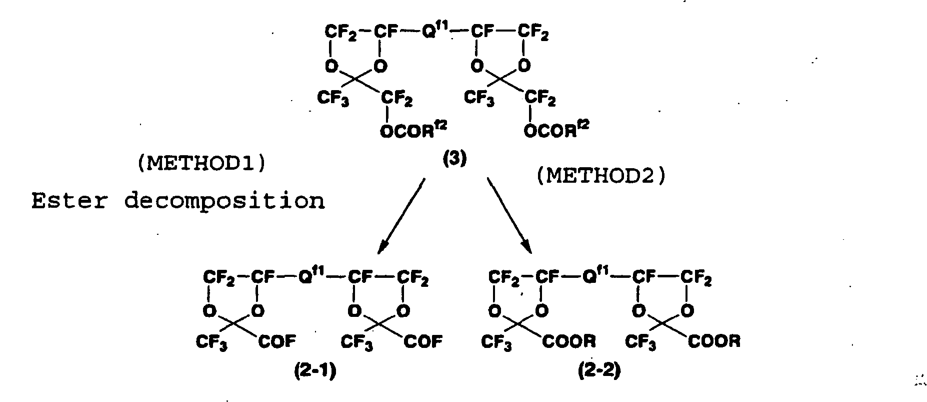

In the above formulae, the symbols have the same meanings as mentioned above.

The Methods 1 and 2 may be carried out by a known technique for a decomposition reaction of an ester bond. For example, it is preferred to employ a method of heating the compound (3) in a gas phase or a liquid phase, or a method of heating it in the presence of a nucleophilic or electrophilic agent. In either method, the reaction temperature is preferably from 50 to 300°C, more preferably from 100 to 250°C.

In the method of heating in the presence of a nucleophilic or electrophilic agent in the Method 1, a solvent may or may not be used. It is preferred not to use a solvent, whereby a trouble of separating the solvent can be omitted. Even in a case where no solvent is used, the compound (3) may function as a solvent.

It is preferred to use a nucleophilic agent capable of generating fluorine ions, as the nucleophilic agent in the Method 1, whereby the reaction temperature for the heat decomposition can be made further lower. In the case where a nucleophilic agent capable of generating fluorine ions is used, the reaction temperature is preferably from -30°C to the boiling point of the compound (2-1). When the reaction is carried out in the vicinity of the boiling point, it is preferred to carry out the reaction while the product is withdrawn from the reaction system.

As the nucleophilic agent capable of generating fluorine ions, it is preferred to employ an alkali metal fluoride. As such an alkali metal fluoride, NaF, NaHF₂, KF or CsF is preferred. In a case where the decomposition reaction is carried out by means of an alkali metal fluoride, it is assumed that the -CF₂OCOR^{f2} group becomes a -COF group via a -CF₂OM group (wherein M is an alkali metal atom corresponding to the alkali metal fluoride employed).

In the Method 1, a compound represented by the formula R^{f2}-COF will also be formed together with the compound (2-1). Such a compound can be re-used as a compound (8) to be used for the production of a compound (4).

The Method 2 can be carried out by means of a known reaction technique. As the compound represented by the formula R-OH in the Method 2, methanol, ethanol, isopropanol or t-butanol may, for example, be mentioned. The reaction temperature for the reaction is preferably from -30°C to the boiling point of the compound represented by the formula R-OH.

Then, the compound (2) obtained by the above Method 1 or 2 is thermally decomposed to obtain a compound (1A) and/or a compound (1B). As a method for the heat decomposition, it is preferred to employ the following Method 3 or 4.

Method 3: A method wherein the compound (2-1) obtained by the Method 1 is heated for heat decomposition to obtain a compound (1A) and/or (1B).

Method 4: A method wherein the compound (2-1) obtained by the Method 1 or the compound (2-2) obtained by the Method 2 is reacted with an alkali metal hydroxide to convert X² to a -OM group (wherein M represents an alkali metal atom corresponding to the alkali metal hydroxide employed) and then, heated for heat decomposition to obtain a compound (1A) and/or a compound (1B).

The heat decomposition reaction in the Method 3 may be carried out in a gas phase or in a liquid phase, and it is efficient and thus preferred to carry out the reaction in a gas phase. More specifically, to carry out the reaction in a gas phase, a tubular reactor filled with glass beads, an alkali metal salt or an alkaline earth metal salt, is prepared. Then, the compound (2-1) obtained by the Method 1 is permitted to flow through the reactor in a gas state, and a formed gas containing the compound (1A) and/or the compound (1B) is condensed for recovery. The compound (2-1) is preferably permitted to flow together with an inert gas. The reaction temperature for such a reaction is preferably from 150 to 500°C, particularly preferably from 200 to 350°C.

The content of the compound (1A) and/or the compound (1B) in the product can be suitably changed by adjusting the reaction conditions, and from the viewpoint of usefulness, it is preferred that the compound (1A) will be the main product. The Method 3 which is carried out in a gas phase, is more suitable for an industrial production process than the Method 4.

As the alkali metal hydroxide in the Method 4, NaOH or KOH is preferred. Further, the amount of alkali metal hydroxide is preferably from 0.95 to 1.05 mol, particularly preferably from 1.00 to 1.05 mol, relative to the amount of the compound (2-2).

The temperature for the reaction with the alkali metal hydroxide is preferably from -30°C to the boiling point of the solvent. Such a reaction is preferably carried out in the presence of a solvent. As the solvent, methanol, ethanol, isopropanol or t-butanol may, for example, be mentioned.

The temperature for the heat decomposition is preferably from 150 to 400°C, particularly preferably from 150 to 300°C. The Method 4 is a method advantageous for a compound having a low stability against heat, since the reaction can be carried out at a temperature lower than the Method 3.

In the above production methods, the compound (1A) will be formed as a single compound irrespective of whether the structure of Q^{f1} is a symmetric structure or an asymmetric structure. However, in a case where the structure of Q^{f1} is an asymmetric structure, the compound (1B) may be formed as two types of compounds. When a trouble of separating the products, etc. are taken into consideration, the structure of Q^{f1} is preferably a symmetric structure, and Q¹ is preferably selected to be a group having a structure which becomes a symmetric structure when converted to Q^{f1}.

The compounds (1A) and (1B) synthesized as described above, are useful as monomers which become raw materials for fluorinated polymers, and the compound (1A) is a novel compound which is useful also as a crosslinking agent.

In a case where it is used as a crosslinking agent, the proportion of the structural units corresponding to the compound (1A) and the structural units corresponding to the compound (1B) in the crosslinked compound is made to be preferably at least 10 mass%, particularly preferably at least 20 mass%. When the proportion is at least 10 mass%, the heat resistance and the mechanical properties at high temperatures of the crosslinked product can be more improved.

The polymer of the present invention is a novel polymer essentially comprising at least one type of repeating units selected from a repeating unit represented by the following formula (9), a repeating unit represented by the following formula (10) and a repeating unit represented by the following formula (11). When the compound (1A) is polymerized, a polymer having repeating units (9) and/or repeating units (10) will be formed. Further, when the compound (1B) is polymerized, a polymer essentially comprising repeating units (11) will be formed. Namely, the novel polymer of the present invention can be produced by polymerizing the compound (1A) and/or the compound (1B).

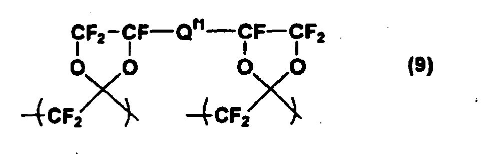

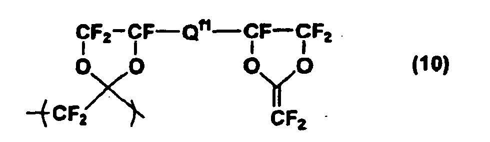

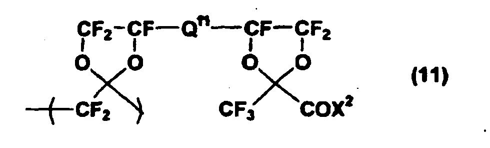

In the above formulae, the symbols have the same meanings as mentioned above.

The novel polymer may be a polymer consisting solely of at least one type of repeating units selected from the repeating units (9), the repeating unit (10) and the repeating units (11), or a polymer containing repeating units other than such repeating units (hereinafter referred to as other repeating units). When the polymer contains other repeating units, the content of such other repeating units may optionally be changed depending upon the particular application of the polymer. Usually, the content of such other repeating units in the polymer is preferably from 1 to 99 mass%.

As such repeating units, repeating units obtained by polymerizing the following polymerizable compounds (hereinafter sometimes referred to as other polymerizable compounds), are preferred. An ethylenic monomer such as tetrafluoroethylene, hexafluoropropylene, trifluoroethylene, chlorotrifluoroethylene, vinylidene fluoride, vinyl fluoride or ethylene; a fluorinated vinyl ether such as perfluoro(methyl vinyl ether), perfluoro-(propyl vinyl ether), perfluoro(2,5-dimethyl-3,6-dioxa-1-nonene), 4H,4H-perfluoro(propyl vinyl ether), methylperfluoro(5-oxa-6-hexenoate) or perfluoro(4-methyl-3,6-dioxa-7-octyl) sulfonylchloride; a cyclopolymerizable monomer such as perfluoro(allyl vinyl ether), perfluoro(butenyl vinyl ether), perfluoro(4-methyl-3-oxa-1,6-heptadiene), perfluoro(3,5-dioxa-1,6-heptadiene), 1,1,2,4,4,5,5-heptafluoro-3-oxa-heptadiene or 1,1,2,4,5,5-hexafluoro-4-trifluoromethyl-3-oxa-1,6-heptadiene; perfluoro(2,2-dimethyl-1,3-dioxol), perfluoro(4-methoxy-1,3-dioxol), perfluoro(2-methylene-4-methyl-1,3-dioxolan), perfluoro(2-methylene-4-propyl-1,3-dioxolan), etc.

The polymerization reaction of the compound (1A) and/or the compound (1B) is carried out preferably by a radical polymerization reaction, more preferably by a thermal polymerization reaction or a photo polymerization reaction. In the case of the thermal polymerization reaction, as a polymerization initiator, a known organic peroxide, azo compound or persulfate may, for example, be employed. Further, in the case of the photo polymerization reaction, an acetophenone type or benzoinmethyl ether type photo polymerization initiator may, for example, be employed. The proportion of polymerization initiator to be used is preferably from 0.01 to 1 mass% based on the total amount of polymerizable monomers to be used for the polymerization reaction.

As the polymerization method, various known methods may be employed, such as bulk polymerization, solution polymerization, emulsion polymerization, suspension polymerization or polymerization in a supercritical fluid. Further, the compound (1A) of the present invention is a thermally curable polymerizable compound, and accordingly, it may be cast in a mold having a desired shape, on a substrate or on a support film, followed by the polymerization reaction.

The temperature for the polymerization reaction is not particularly limited, and the polymerization is preferably carried out at a temperature where the half-life period of the polymerization initiator will be from about 3 to 10 hours. It is preferably from about 15 to 150°C. The polymerization can be carried out under any condition of normal pressure, elevated pressure or reduced pressure. As a solvent to be used for carrying out a solution polymerization, it is preferred to employ a solvent having a boiling point of from 20 to 350°C, since the handling is easy, more preferably a solvent having a boiling point of from 40 to 150°C. In the case of bulk polymerization, it is preferred to carry it out in a closed system, so that evaporation of monomers can be suppressed even under a high temperature condition. Whereas, if the polymerization is to be carried out in an open system, it is preferred to carry it out at a temperature lower than the boiling point of the monomers. Further, in a case where it is desired to obtain a transparent optical resin shaped product by bulk polymerization, as a polymerization initiator, it is preferred to employ a polymerization initiator made of a fluorinated peroxide compound such as perfluorobenzoyl peroxide, perfluorodibutyryl peroxide or perfluoro(t-butyl peroxide).

The proportion of the compound (1A) and the compound (1B) to be used, may be suitably selected taking the particular application or the like into consideration. For example, with respect to the amount of the compound (1A) and/or the compound (1B) in the case of an application to e.g. an optical resin material for e.g. optical waveguides, lenses and transparent sealing agents, the total amount of the compound (1A) and the compound (1B) is preferably from 0.1 to 70 mass%, more preferably from 1 to 50 mass%, based on the mass of all polymerizable compounds to be used. The amount of other polymerizable compounds is preferably from 99.9 to 30 mass%, particularly preferably from 99 to 50 mass%, based on the mass of all polymerizable compounds.

Of the polymer of the present invention, the lower limit of the mass average molecular weight is preferably 10,000, more preferably 50,000. By adjusting the lower limit of the mass average molecular weight to be 10,000, it is possible to improve the mechanical strength. Further, the upper limit of the mass average molecular weight is preferably 1,000,000 from the viewpoint of the solubility in a solvent or melt-moldability. The mass average molecular weight can be adjusted by e.g. the amounts of the polymerization initiator and the chain transfer agent relative to the monomers.

The following repeating unit (13) may be contained in the polymer obtained by the polymerization of the compound (1A). The repeating unit (13) may be formed by cyclopolymerization of two double bonds in the compound (1A). The structure of this repeating unit may be influential over the function or the yield of the polymer and should preferably be at most 10 mol% in the polymer.

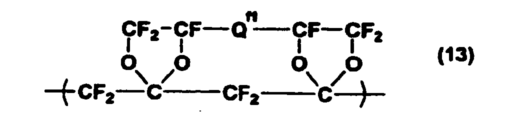

In the above formula, Q^{f1} has the same meaning as mentioned above.

Further, the polymer of the present invention may have a repeating unit represented by the following formula (11')(hereinafter referred to as the repeating unit (11')). The polymer having the repeating unit (11') can be obtained by a method wherein a polymer containing the repeating unit (11) is reacted with a compound represented by the formula R¹-OH (wherein R¹ is a monovalent organic group, particularly preferably an alkyl group which may contain an etheric oxygen atom between carbon-carbon atoms, or a group having hydrogen atoms of such an alkyl group fluorinated), or a method wherein the compound (1B) is reacted with a compound represented by the formula R¹-OH (wherein R¹ is as defined above) to obtain the following compound (1B') which is then polymerized.

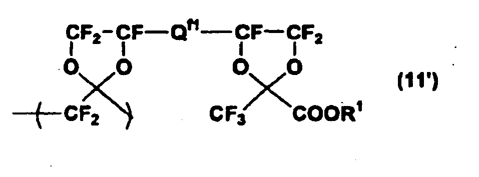

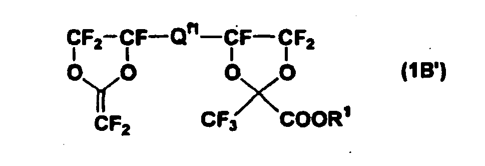

The polymer provided by the present invention is excellent in characteristics such as heat resistance, mechanical strength, transparency and rigidity. The reason for such excellent heat resistance, mechanical strength and rigidity is considered to be such that it has two cyclic structures in a repeating unit. Further, a reason for excellent transparency is considered to be such that the cyclic structures constitute an irregularly linked polymer chain, whereby the polymer tends to be hardly crystallizable and tends to be amorphous.

The polymer to be provided by the present invention may be obtained as an amorphous and transparent polymer. Such a polymer is useful as an optical resin material for optical waveguides, lenses, transparent sealing agents, etc. When used as an optical resin material, it has a merit in that it has transparency and heat resistance. Further, the polymer of the present invention is useful also as a coating agent to form a layer such as an antireflection layer, a non-adhesive layer or an anticorrosion protective layer.

According to the present invention, it is possible to provide a novel fluorinated dioxolan compound useful as a crosslinking agent or a monomer for a fluorinated polymer excellent in characteristics such as heat resistance, mechanical strength, transparency and rigidity. Further, according to the present invention, it is possible to provide a novel compound useful as an intermediate for the production of a novel fluorinated dioxolan compound. Further, according to the present invention, it is possible to provide a novel fluorinated polymer excellent in characteristics such as heat resistance, mechanical strength, transparency and rigidity.

### EXAMPLES

Now, the present invention will be described in further detail with reference to Examples. However, it should be understood that the present invention is by no means restricted to such Examples. Here, R-113 represents 1,1,2-trichloro-1,2,2-trifluoroethane. GC represents gas chromatography, and GC-MS represents gas chromatography mass spectrometry. Further, the GC purity is a purity obtained from the peak area ratio in the GC analysis. The pressure in Examples will be shown by an absolute pressure unless otherwise specified.

### EXAMPLE 1: Preparation of compound (4a)

### EXAMPLE 1-1: Preparation 1 of compound (4a)

CH₃C(O)CH₂OH (59 g), compound (6a) (60 g), methyl orthoformate (84.3 g) and p-toluensulfonic acid (3.1 g) are put into a 1 L flask and heated at 80°C for 3 hours with stirring. Then, the pressure in the flask is reduced to 2.5 mbar to remove methanol and methyl formate as low boiling point components thereby to obtain compound (5a) with M/e being 278 by GC-MS in a GC purity of about 65%.

Then, to this reaction solution, NaF (91 g) is added with stirring under cooling with ice, and then, the following compound (8a) (240 g) is dropwise added over a period of 2 hours. After stirring for further 2 hours, the mixture is stirred at room temperature over night. This reaction solution is purified by a silica gel column by means of a developing solvent of ethyl acetate/hexane=1/2 (mass ratio) and further distilled under reduced pressure to obtain a diacetal compound of the following formula (4a) (205 g) in a GC purity of 99% as a distillate of from 173 to 176°C at 2 mbar.

### EXAMPLE 1-2: Preparation 2 of compound (4a)

CH₃C(O)CH₂OH (59 g), the following compound (6a) (60 g), methyl orthoformate (84.3 g) and p-toluenesulfonic acid (3.1 g) were put into a 1 L flask and heated at 80°C for 3 hours with stirring. Then, the pressure in the flask was reduced to 2.5 mbar to remove methanol and methyl formate as low boiling point components thereby to obtain compound (5a) (155 g) with M/e being 278 by GC-MS in a GC purity of about 65%.

Then, to this reaction solution, NaF (91 g) was added with stirring under cooling with ice, and then, the following compound (8a) (240 g) was dropwise added over a period of 2 hours. After stirring for further 2 hours, the mixture was stirred at 25°C for 12 hours. This reaction solution was purified by a silica gel column by means of a developing solvent of ethyl acetate/hexane=1/2 (mass ratio) and further distilled under reduced pressure to obtain a diacetal compound of the following formula (4a) (205 g) in a GC purity of 99% as a distillate of from 173 to 176°C at 2 mbar.

¹H-NMR of compound (5a) (282.7 MHz, solvent: (CD₃)₂CO, standard: TMS) δ(ppm): 1.26 (6H), 3.4 (2H), 3.5 to 3.8 (10H), 4.0 to 4.3 (4H).

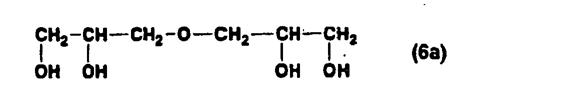

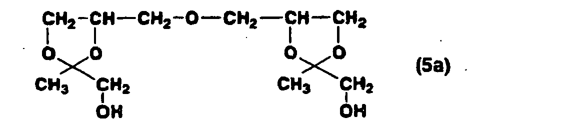

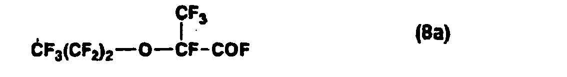

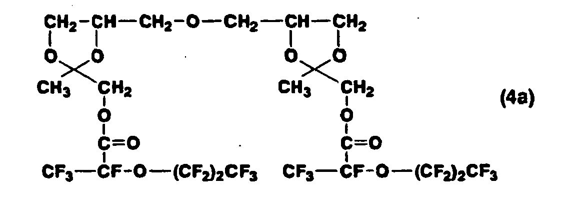

### EXAMPLE 2: Preparation 3 of compound (4a)

The following compound (8a) (365 g) was put into a 1 L flask, and NaF (124 g) was added with stirring under cooling with ice. Then, while the internal temperature of the flask was maintained to be at most 10°C, CH₃C(O)CH₂OH (74 g) was dropwise added over a period of 3 hours, followed by stirring for further 2 hours. The internal temperature of the flask was raised to room temperature, and stirring was further continued over night. Then, the solution was diluted with dichloropentafluoropropane ("AK225" trade name, manufactured by Asahi Glass Company, Limited) and then filtered through a polytetrafluoroethylene filter having a pore size of 0.5 µm to remove NaF, the filtrate was distilled under reduced pressure to obtain the following compound (7a) (307 g) in a GC purity of 96%.

Then, compound (7a) (307 g), compound (6a) (60 g), methyl orthoformate (84.3 g) and p-toluensulfonic acid (3.1 g) were put into a 1 L flask and heated at 80°C for 3 hours with stirring. Then, the pressure in the flask was reduced to 2 mbar to remove methanol and methyl formate as low boiling point components. The obtained crude liquid was purified by a silica gel column by means of a developing solvent of ethyl acetate/hexane=1/2 (mass ratio) and further distilled under reduced pressure at from 173 to 176°C at 2 mbar. The distillate was analyzed by ¹H-NMR and confirmed to be a diacetal compound represented by the following formula (4a). The yield of the compound (4a) was 228 g, and the GC purity was 99%.

¹H-NMR (282.7 MHz, solvent: CDCl₃, standard: TMS) δ(ppm): 1.4 (6H), 3.5 to 3.6 (4H), 3.7 to 4.2 (4H), 4.3 to 4.5 (6H).

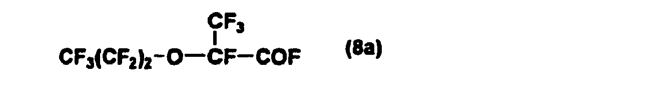

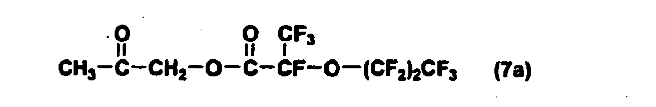

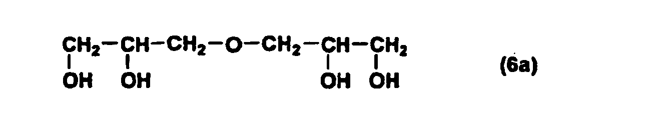

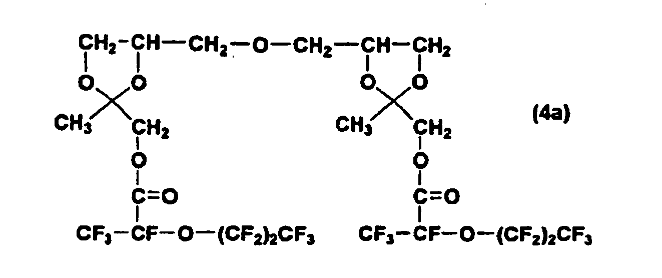

### EXAMPLE 3: Preparation of compound (3a)

Into a 3,000 mL autoclave made of nickel, R-113 (1,700 g) was put and stirred, and the temperature in the autoclave was maintained to be at 25°C. At the gas outlet portion of the autoclave, a condenser held at 20°C, a NaF pellet-packed layer and a condenser held at -10°C were installed in series. Further, a liquid-returning line was installed to return a condensed liquid from the condenser held at -10°C to the autoclave. After blowing nitrogen gas into the autoclave at room temperature for 1 hour, a fluorine gas diluted to 20% with nitrogen gas (hereinafter referred to as a 20% fluorine gas) was further supplied at room temperature at a flow rate of 17.04 L/hr for 1 hour. Then, while the 20% fluorine gas was blown at the same flow rate, a solution having the compound (4a) obtained in Example 2 (110 g) dissolved in R-113 (800 g), was injected over a period of 24 hours.

Then, while the 20% fluorine gas was supplied at the same flow rate, the pressure in the autoclave was raised to 0.15 MPa, and a R-113 solution having a benzene concentration of 0.01 g/mL was injected in an amount of 30 mL while the temperature was raised to from 25°C to 40°C. Then, the inlet for the benzene solution of the autoclave was closed, and stirring was continued for 0.3 hour.

Then, while the pressure in the autoclave was maintained to be 0.15 MPa and the temperature in the autoclave was maintained to be at 40°C, the above mentioned benzene solution was injected in an amount of 20 mL, whereupon the benzene solution injection inlet of the autoclave was closed, and stirring was continued for 0.3 hour. Further, 20 mL of R-113 was supplied so that the benzene solution in the piping was all injected into the autoclave. The total amount of benzene injected was 0.5 g, and the total amount of R-113 injected was 49 mL.

Further, while a 20% fluorine gas was continuously blown into the autoclave at the same flow rate, stirring was continued for 1 hour. Then, the inner pressure of the autoclave was adjusted to atmospheric pressure, and nitrogen gas was supplied for 1 hour. The product was analyzed by ¹⁹F-NMR, whereby the yield of the following compound (3a) was 92%.

¹⁹F-NMR (282.7 MHz, solvent: CDCl₃, standard: CFCl₃) δ (ppm): -77.8 (2F), -80.5 (8F), -81.1 to -83.7 (18F), -86.0 to -88.0 (6F), -122.4 (2F), -130.2 (4F), -132.2 (2F).

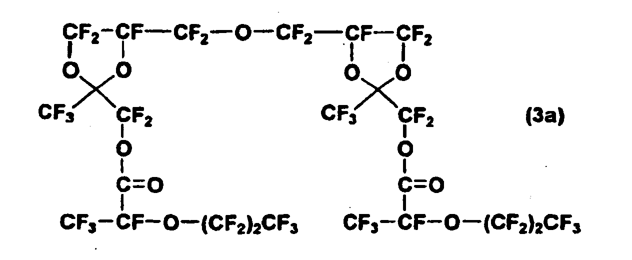

### EXAMPLE 4: Preparation of compound (2-1a)

### EXAMPLE 4-1: Preparation 1 of compound (2-1a)

The compound (3a) (135.8 g) obtained in Example 3 is charged into a flask together with sufficiently dried KF powder (1.2 g) and heated at 40°C with stirring. After reacting the mixture for 2 hours under reflux, low boiling components are distilled off. After cooling, a sample (57 g) recovered from the flask is filtered to recover a liquid sample. By NMR, it is confirmed that the following compound (2-1a) is formed as the main product. The yield is about 94%, and the GC purity is about 95%.

### EXAMPLE 4-2: Preparation 2 of compound (2-1a)

The following compound (3a) (126.2 g) obtained in Example 3 was charged into a flask together with sufficiently dried KF powder (1.2 g) and heated at 40°C with stirring. After reacting the mixture for 2 hours under reflux, low boiling components were distilled off. After cooling, a sample (57 g) recovered from the flask was filtered to recover a liquid sample. By NMR, it was confirmed that the following compound (2-1a) was formed as the main product. The yield was about 95%, and the GC purity was about 99%.

¹⁹F-NMR of compound (2-1a) (282.65 MHz, solvent: CDCl₃, standard: CFCl₃) δ (ppm): 23.8 (2F), -78.1 to 79.4 and -81.7 to -84.4 (8F), -82.0 (6F), -123.9 (2F).

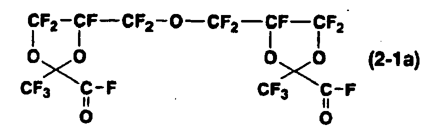

### EXAMPLE 5: Preparation of compound (2-2a)

Methanol (140 g) was put into a 500 mL polyethylene bottle, and the compound (3a) (140 g) obtained in Example 3 was dropwise added with stirring under cooling with ice. After completion of the dropwise addition, stirring was continued at room temperature over night. Low boiling components in a crude liquid were distilled off by means of a rotary evaporator, followed by washing with water 3 times, to obtain a diester compound represented by the following formula (2-2a) (67 g) in a GC purity of 92%.

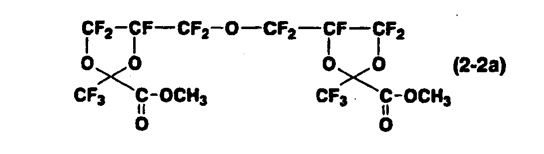

¹⁹F-NMR of compound (2-1a) (282.65 MHz, solvent: CDCl₃, standard: CFCl₃) δ (ppm) : -78.3 and -83.8 (4F), -81.5 (6F), -82 to -84 (4F), -123.6 (2F).

### EXAMPLE 6: Preparation of compound (1-1) and compound (1-3)

Into a 300 mL Erlenmeyer flask, the diester compound (2-2a) (22 g) obtained in Example 5 and phenolphthalein were put, and a solution of KOH/methanol=1/5 (mass ratio) was dropwise added with stirring at room temperature. When 167 g of the solution was added, the color became red. Methanol was distilled off by means of a rotary evaporator, followed by vacuum drying at 100°C for one day to obtain a solid substance (28 g).

Then, the solid substance was put into a 100 mL flask and heated at a temperature of from 250 to 280°C under vacuum by a vacuum pump. A heat decomposition product generated as a gas was collected in a trap tube cooled by dry ice-ethanol. The collected liquid was analyzed by GC-MS, whereby the following compounds (1-1) to (1-5) were contained. M/e of the compound (1-1) was 466 (purity: 63%), M/e of the compound (1-2) was 544, M/e of the compound (1-3) was 532, M/e of the compound (1-4) was 486, and M/e of the compound (1-5) was 504.

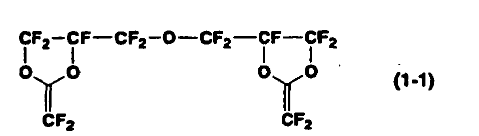

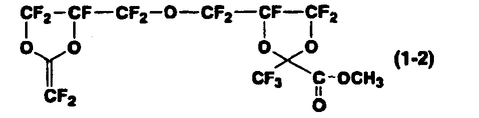

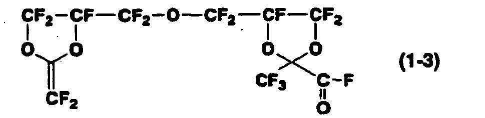

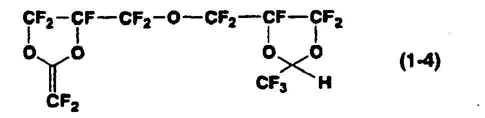

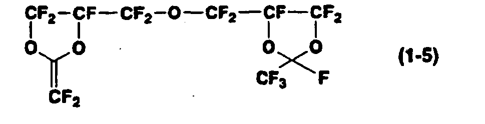

The compound (1-3) was methyl-esterified to the compound (1-2), and then, the mixture was distilled to separate the compound (1-1) and the compound (1-2). The fraction of 44°C/5 mbar contained 94% of the compound (1-1). This fraction further contained 0.5% of the compound (1-2), 4.8% of the compound (1-4) and 0.4% of the compound (1-5). Further, the fraction of 69°C/2 mbar contained 97% of the compound (1-2). This fraction further contained 0.1% of the compound (1-4).

The ¹⁹F-NMR spectrum and ¹³C-NMR spectrum of the compound (1-1) were as follows.

¹⁹F-NMR (564.55 MHz, solvent: CDCl₃, standard: CFCl₃) δ (ppm): -80 to -84 (4F), -82.8 and -88.3 (4F), -126.0 and -127.3 (4F) -128.8 (2F).

¹³C-NMR (150.80 MHz, solvent and standard: CDCl₃) δ (ppm): 104.9, 116.4, 122.2, 133.8, 142.9.

The ¹⁹F-NMR spectrum of the compound (1-2) was as follows.

¹⁹F-NMR (282.65 MHz, solvent: CDCl₃, standard: CFCl₃) δ (ppm): -80 to -84 (4F), -82.8 and -88.3 (2F), -78.3 and -83.8 (2F), -81.5 (3F), -123.6 (1F), 125.2 and 126.7 (2F), -128.6 (1F).

### EXAMPLES 7: Preparation 1 of polymer

The compound (1-1) (5 g) obtained in Example 6 and perfluorobenzoyl peroxide (0.01 g) were put into a glass tube and cooled and solidified by liquid nitrogen, followed by vacuum deaeration. After carrying out deaeration 3 times by repeating thawing and freezing, the glass tube was sealed and heated for 15 hours in an oven of 65°C. After cooling, a polymer was taken out from the glass tube, and it was a colorless transparent glassy solid. The weight after vacuum drying at 100°C was 3.8 g. Then, using TMA (thermo mechanical analyzer), the softening point of a polymer specimen was measured by an indentation method by means of a quartz probe, but within a range of from room temperature to 300°C, no distinct softening point was observed. Further, TGA (thermo gravimetric analysis) was carried out at a temperature raising rate of 10°C/min in a nitrogen atmosphere, whereby the mass reduction gradually took place from about 260°C, and the 10% mass reduction temperature was 420°C. The refractive index of the glassy solid as measured by an Abbe refractometer was 1.355.

### EXAMPLE 8: Preparation 2 of polymer

The compound (1-2) (2.5 g) obtained in Example 6 and perfluorobenzoyl peroxide (5 mg) were put into a glass tube and cooled and solidified by liquid nitrogen, followed by vacuum deaeration. After carrying out deaeration 3 times by repeating thawing and freezing, the glass tube was sealed and heated at 70°C for 6 hours and further at 90°C for 2 hours. After cooling, the polymer was taken out from the glass tube and further vacuum-dried over night at 100°C. A colorless transparent glassy solid was obtained in an amount of 2.1 g. The refractive index of the glassy solid as measured by an Abbe refractometer was 1.355. The measurement by a differential scanning calorimetry (DSC) was carried out in a nitrogen atmosphere at a temperature raising rate of 30°C/min, whereby the glass transition temperature was observed at 71°C. Further, TGA (thermo gravimetric analysis) was carried out in a nitrogen atmosphere at a temperature raising rate of 10°C/min, whereby a mass reduction gradually took place from about 250°C, and the 10% mass reduction temperature was 400°C.

### EXAMPLE 9: Preparation 3 of polymer

The compound (1-1) (2.5 g) obtained in Example 6 and perfluorobutenyl vinyl ether (2.5 g) and perfluoroebenzoyl peroxide (0.01 g) were put into a glass tube and cooled and solidified by liquid nitrogen, followed by vacuum deaeration. After carrying out deaeration 3 times by repeating thawing and freezing, the glass tube was sealed and heated for 18 hours in an oven of 60°C and then heated in the order of 70°C, 90°C and 110°C for 1 hour each. After cooling, the polymer was taken out from the glass tube and further vacuum-dried at 100°C over night. A colorless transparent glassy solid was obtained in an amount of 3.2 g. The refractive index of the glassy solid as measured by an Abbe refractometer was 1.355.

The measurement by a differential scanning calorimetric was carried out in a nitrogen atmosphere at a temperature raising rate of 30°C/min, whereby within a range of from 40 to 300°C, no heat absorption due to a glass transition temperature was observed. Further, using TMA, the softening temperature was measured at a temperature raising rate of 10°C/min in air, whereby within a range of from room temperature to 300°C, no abrupt change in the expansion coefficient due to a softening temperature was observed.

The glass transition temperature of a homopolymer of known perfluorobutenyl vinyl ether is 108°C by DSC. Thus, the heat resistance was found remarkably improved by copolymerizing the compound (1-1) with perfluorobutenyl vinyl ether.

### INDUSTRIAL APPLICABILITY

The present invention provides a novel fluorinated polymer having improved characteristics such as heat resistance, mechanical strength, transparency and rigidity, and a novel fluorinated dioxolan compound useful as e.g. a crosslinking agent or a raw material for such a fluorinated polymer, the polymer and a method for producing such a polymer.

## Claims

1. A compound represented by the following formula (1A) (wherein Q^{f1} represents a single bond, an oxygen atom, a C₁₋₅ perfluoroalkylene group, or a C₁₋₅ perfluoroalkylene group having a etheric oxygen atom inserted between carbon-carbon atoms):

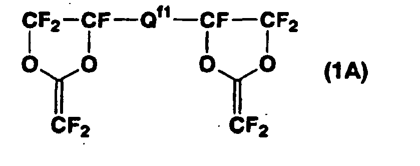

2. A compound represented by the following formula (1B) (wherein Q^{f1} represents a single bond, an oxygen atom, a C₁₋₅ perfluoroalkylene group, or a C₁₋₅ perfluoroalkylene group having a etheric oxygen atom inserted between carbon-carbon atoms, and X² is a fluorine atom, or a group represented by -OR (wherein R is a hydrogen atom, a C₁₋₅ alkyl group, or a C₁₋₅ alkyl group having an etheric oxygen atom inserted between carbon-carbon atoms)):

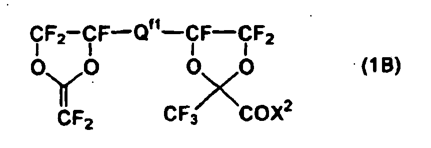

3. A compound represented by the following formula (5) (wherein Q¹ represents a single bond, an oxygen atom, a C₁₋₅ alkylene group, or a C₁₋₅ alkylene group having an etheric oxygen atom inserted between carbon-carbon atoms):

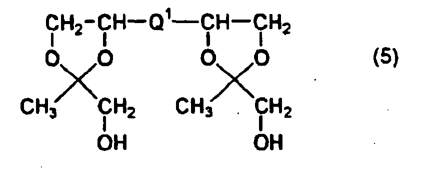

4. A compound represented by the following formula (4) (wherein Q¹ represents a single bond, an oxygen atom, a C₁₋₅ alkylene group, or a C₁₋₅ alkylene group having an etheric oxygen atom inserted between carbon-carbon atoms, and two R^{f2} in the formula may be the same or different, and each represents a C₁₋₁₀ perfluoroalkyl group, or a C₁₋₁₀ perfluoroalkyl group having an etheric oxygen atom inserted between carbon-carbon atoms):

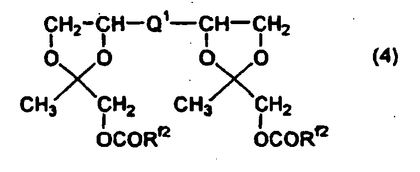

5. A compound represented by the following formula (3) (wherein Q^{f1} represents a single bond, an oxygen atom, a C₁₋₅ perfluoroalkylene group, or a C₁₋₅ perfluoroalkylene group having a etheric oxygen atom inserted between carbon-carbon atoms, and two R^{f2} in the formula may be the same or different, and each represents a C₁₋₁₀ perfluoroalkyl group, or a C₁₋₁₀ perfluoroalkyl group having an etheric oxygen atom inserted between carbon-carbon atoms):

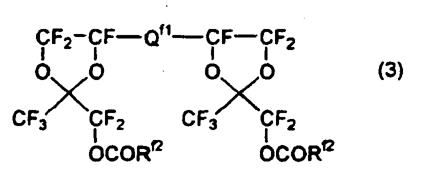

6. A compound represented by the following formula (2) (wherein Q^{f1} represents a single bond, an oxygen atom, a C₁₋₅ perfluoroalkylene group, or a C₁₋₅ perfluoroalkylene group having a etheric oxygen atom inserted between carbon-carbon atoms, and two X² in the formula may be the same or different, and each represents a fluorine atom, or a group represented by -OR (wherein R represents a hydrogen atom, a C₁₋₅ alkyl group, or a C₁₋₅ alkyl group having an etheric oxygen atom inserted between carbon-carbon atoms)):

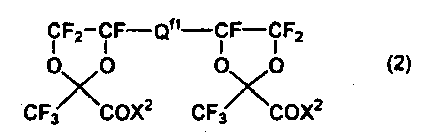

7. A polymer essentially comprising at least one type of repeating units selected from a repeating unit represented by the following formula (9), a repeating unit represented by the following formula (10) and a repeating unit represented by the following formula (11) (wherein Q^{f1} represents a single bond, an oxygen atom, a C₁₋₅ perfluoroalkylene group, or a C₁₋₅ perfluoroalkylene group having a etheric oxygen atom inserted between carbon-carbon atoms, and, in a case where the polymer essentially comprises at least two types of repeating units, Q^{f1} in the formulae may be the same or different, X² represents a fluorine atom, or a group represented by -OR (wherein R represents a hydrogen atom, a C₁₋₅ alkyl group, or a C₁₋₅ alkyl group having an etheric oxygen atom inserted between carbon-carbon atoms)):

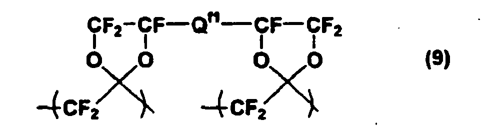

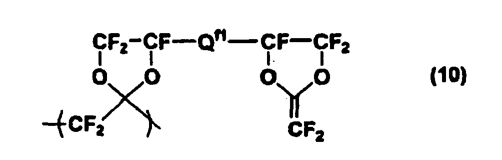

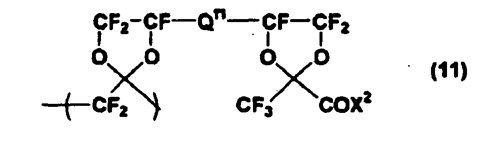

8. A method for producing a polymer, **characterized by** polymerizing a compound represented by the following formula (1A) and/or a compound represented by the following formula (1B), or polymerizing a compound represented by the following formula (1A) and/or a compound represented by the following formula (1B), and another polymerizable compound (wherein Q^{f1} represents a single bond, an oxygen atom, a C₁₋₅ perfluoroalkylene group or a C₁₋₅ perfluoroalkylene group having a etheric oxygen atom inserted between carbon-carbon atoms, and in a case where the polymer essentially comprises at least two types of repeating units, Q^{f1} in the formulae may be the same or different, X² represents a fluorine atom or a group represented by -OR (wherein R represents a hydrogen atom, a C₁₋₅ alkyl group, or a C₁₋₅ alkyl group having an etheric oxygen atom inserted between carbon-carbon atoms)):

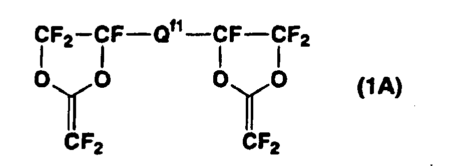

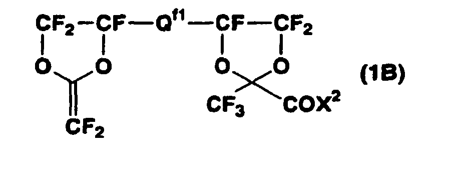

## Patentansprüche

1. Verbindung, dargestellt durch die folgende Formel (1A) (worin Q^{f1} eine Einfächbindung, ein Sauerstoffatom, eine C₁₋₅ Perfluoralkylengruppe oder eine C₁₋₅ Perfluoralkylengruppe mit einem Ethersauerstoffatom, insertiert zwischen Kohlenstoff-Kohlenstoffatome, darstellt):

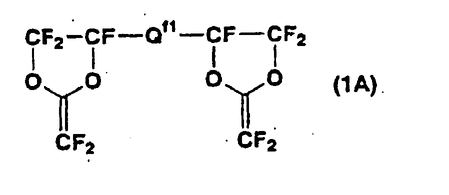

2. Verbindung, dargestellt durch die folgende Formel (1 B) (worin Q^{f1} eine Einfachbindung, ein Sauerstoffatom, eine C₁₋₅ Perfluoralkylengruppe oder eine C₁₋₅ Perfluoralkylengruppe mit einem Ethersauerstoffatom, insertiert zwischen Kohlenstoff-Kohlenstoffatome, darstellt, und X² ein Fluoratom oder eine Gruppe ist, dargestellt durch -OR (worin R ein Wasserstoffatom, eine C₁₋₅ Alkylgruppe oder eine C₁₋₅ Alkylgruppe mit einem Ethersauerstoffatom, insertiert zwischen Kohlenstoff-Kohlenstoffatome, ist)):

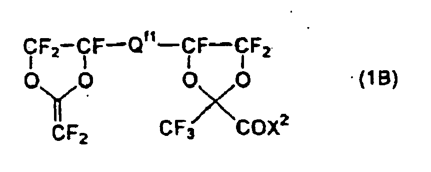

3. Verbindung, dargestellt durch die folgende Formel (5) (worin Q¹ eine Einfachbindung, ein Sauerstoffatom, eine C₁₋₅ Alkylengruppe oder eine C₁₋₅ Alkylengruppe mit einem Ethersauerstoffatom, insertiert zwischen Kohlenstoff-Kohlenstoffatome, darstellt):

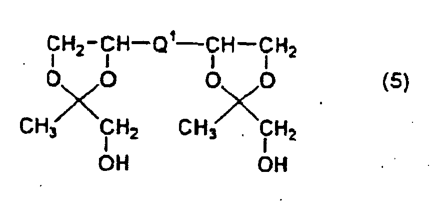

4. Verbindung, dargestellt durch die folgende Formel (4) (worin Q¹ eine Einfachbindung, ein Sauerstoffatom, eine C₁₋₅ Alkylengruppe oder eine C₁₋₅ Alkylengruppe mit einem Ethersauerstoffatom, insertiert zwischen Kohlenstoff-Kohlenstoffatome, darstellt und zwei Reste R^{f2} in der Formel gleich oder unterschiedlich sein können und jeweils eine C₁₋₁₀ Perfluoralkylgruppe oder eine C₁₋₁₀ Perfluoralkylgruppe mit einem Ethersauerstoffatom, insertiert zwischen Kohlenstoff-Kohlenstoffatome, darstellen):

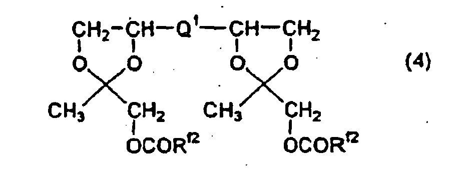

5. Verbindung, dargestellt durch die folgende Formel (3) (worin Q^{f1} eine Einfachbindung, ein Sauerstoffatom, eine C₁₋₅ Perfluoralkylengruppe oder eine C₁₋₅ Perfluoralkylengruppe mit einem Ethersauerstoffatom, insertiert zwischen Kohlenstoff-Kohlenstoffatome, darstellt und zwei Reste R^{f2} in der Formel gleich oder unterschiedlich sein können und jeweils eine C₁₋₁₀ Perfluoralkylgruppe oder eine C₁₋₁₀ Perfluoralkylgruppe mit einem Ethersauerstoffatom, insertiert zwischen Kohlenstoff-Kohlenstoffatome, darstellen):

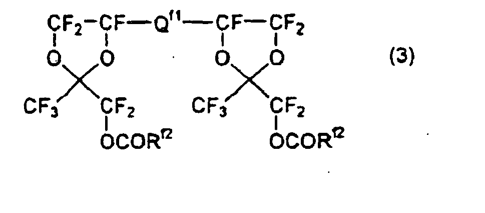

6. Verbindung, dargestellt durch die folgende Formel (2) (worin Q^{f1} eine Einfachbindung, ein Sauerstoffatom, eine C₁₋₅ Perfluoralkylengruppe oder eine C₁₋₅ Perfluoralkylengruppe mit einem Ethersauerstoffatom, insertiert zwischen Kohlenstoff-Kohlenstoffatome, darstellt und zwei X² in der Formel gleich oder unterschiedlich sein können und jeweils ein Fluoratom oder eine Gruppe, dargestellt durch -OR, darstellen (worin R ein Sauerstoffatom, eine C₁₋₅ Alkylgruppe oder eine C₁₋₅ Alkylgruppe mit einem Ethersauerstoffatom, insertiert zwischen Kohlenstoff-Kohlenstoffatome, darstellt)):

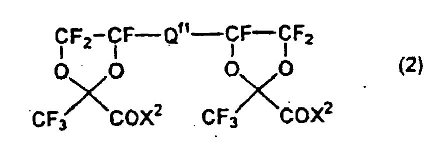

7. Polymer, im wesentlichen umfassend mindestens einen Typ an wiederkehrenden Einheiten, ausgewählt aus einer wiederkehrenden Einheit, dargestellt durch die folgende Formel (9), einer wiederkehrenden Einheit, dargestellt durch die folgende Formel (10), und einer wiederkehrenden Einheit, dargestellt durch die folgende Formel (11) (worin Q^{f1} eine Einfachbindung, ein Sauerstoffatom, eine C₁₋₅ Perfluoralkylengruppe oder eine C₁₋₅ Perfluoralkylengruppe mit einem Ethersauerstoffatom, insertiert zwischen Kohlenstoff-Kohlenstoffatome, darstellt, und in einem Fall, wenn das Polymer im wesentlichen mindestens zwei Typen von wiederkehrenden Einheiten umfaßt, Q^{f1} in den Formeln gleich oder unterschiedlich sein kann, X² ein Fluoratom oder eine Gruppe darstellt, dargestellt durch -OR (worin R ein Wasserstoffatom, eine C₁₋₅ Alkylgruppe oder eine C₁₋₅ Alkylgruppe mit einem Ethersauerstoffatom, insertiert zwischen Kohlenstoff-Kohlenstoffatome, darstellt)):

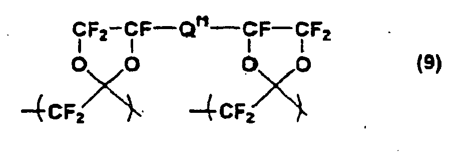

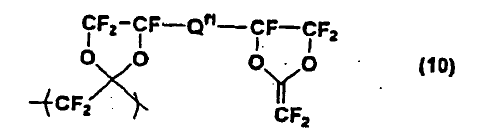

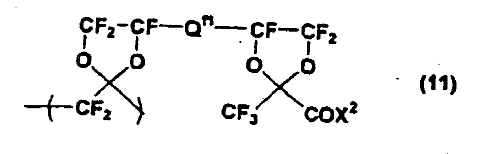

8. Verfahren zur Herstellung eines Polymers, **gekennzeichnet durch** Polymerisieren einer Verbindung, dargestellt **durch** die folgende Formel (1A), und/oder einer Verbindung, dargestellt **durch** die folgende Formel (1 B), oder Polymerisieren einer Verbindung, dargestellt **durch** die folgende Formel (1 A), und/oder einer Verbindung, dargestellt **durch** die folgende Formel (1 B), und einer weiteren polymerisierbaren Verbindung (worin Q^{f1} eine Einfachbindung, ein Sauerstoffatom, eine C₁₋₅ Perfluoralkylengruppe oder eine C₁₋₅ Perfluoralkylengruppe mit einem Ethersauerstoffatom, insertiert zwischen Kohlenstoff-Kohlenstoffatome, darstellt, und in einem Fall, wenn das Polymer im wesentlichen mindestens zwei Typen von wiederkehrenden Einheiten umfaßt, Q^{f1} in den Formeln gleich oder unterschiedlich sein kann, X² ein Fluoratom oder eine Gruppe darstellt, dargestellt **durch** -OR (worin R ein Wasserstoffatom, eine C₁₋₅ Alkylgruppe oder eine C₁₋₅ Alkylgruppe mit einem Ethersauerstoffatom, insertiert zwischen Kohlenstoff-Kohlenstoffatome, darstellt)):

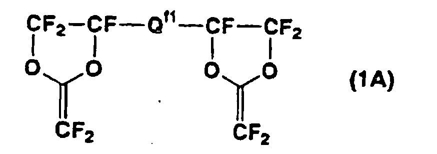

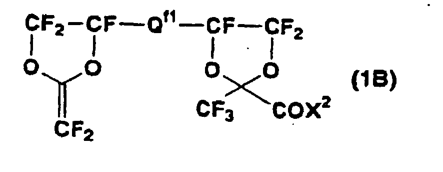

## Revendications

1. Composé représenté par la formule (1A) suivante (dans laquelle Q^{f1} représente une liaison simple, un atome d'oxygène, un groupe perfluoroalkylène en C₁₋₅ ou un groupe perfluoroalkylène en C₁₋₅ ayant un atome d'oxygène d'éther inséré entre des atomes de carbone-carbone) :

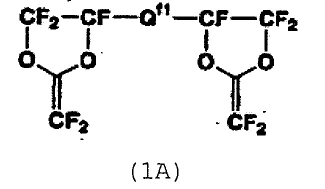

2. Composé représenté par la formule (1B) suivante (dans laquelle Q^{f1} représente une liaison simple, un atome d'oxygène, un groupe perfluoroalkylène en C₁₋₅ ou un groupe perfluoroalkylène en C₁₋₅ ayant un atome d'oxygène d'éther inséré entre des atomes de carbone-carbone, et X² est un atome de fluor, ou un groupe représenté par -OR (où R est un atome d'hydrogène, un groupe alkyle en C₁₋₅, ou un groupe alkyle en C₁₋₅ ayant un atome d'oxygène d'éther inséré entre des atomes de carbone-carbone)) :

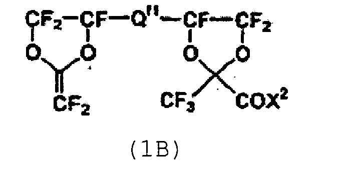

3. Composé représenté par la formule (5) suivante (dans laquelle Q¹ représente une liaison simple, un atome d'oxygène, un groupe alkylène en C₁₋₅, ou un groupe alkylène en C₁₋₅ ayant un atome d'oxygène d'éther inséré entre des atomes de carbone-carbone) :

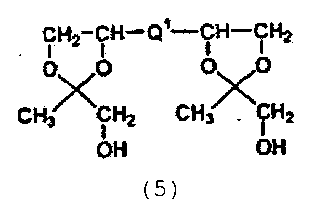

4. Composé représenté par la formule (4) suivante (dans laquelle Q¹ représente une liaison simple, un atome d'oxygène, un groupe alkylène en C₁₋₅, ou un groupe alkylène en C₁₋₅ ayant un atome d'oxygène d'éther inséré entre des atomes de carbone-carbone, et deux R^{f2} dans la formule peuvent être identiques ou différents, et représentent chacun un groupe perfluoroalkyle en C₁₋₁₀, ou un groupe perfluoroalkyle en C₁₋₁₀ ayant un atome d'oxygène d'éther inséré entre des atomes de carbone-carbone) :

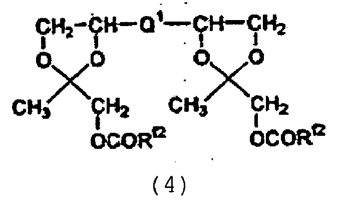

5. Composé représenté par la formule (3) suivante (dans laquelle Q^{f1} représente une liaison simple, un atome d'oxygène, un groupe perfluoroalkylène en C₁₋₅ ou un groupe perfluoroalkylène en C₁₋₅ ayant un atome d'oxygène d'éther inséré entre des atomes de carbone-carbone, et deux R^{f2} dans la formule peuvent être identiques ou différents, et représentent chacun un groupe perfluoroalkyle en C₁₋₁₀, ou un groupe perfluoroalkyle en C₁₋₁₀ ayant un atome d'oxygène d'éther inséré entre des atomes de carbone-carbone) :

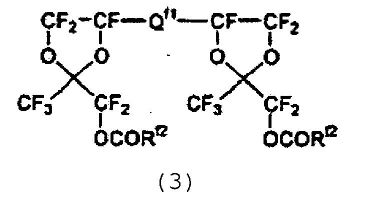

6. Composé représenté par la formule (2) suivante (dans laquelle Q^{f1} représente une liaison simple, un atome d'oxygène, un groupe perfluoroalkylène en C₁₋₅ ou un groupe perfluoroalkylène en C₁₋₅ ayant un atome d'oxygène d'éther inséré entre des atomes de carbone-carbone, et deux X² dans la formule peuvent être identiques ou différents, et représentent chacun un atome de fluor, ou un groupe représenté par -OR (où R est un atome d'hydrogène, un groupe alkyle en C₁₋₅, ou un groupe alkyle en C₁₋₅ ayant un atome d'oxygène d'éther inséré entre des atomes de carbone-carbone)) :

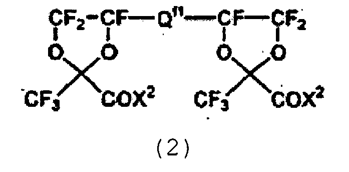

7. Polymère comprenant essentiellement au moins un type de motifs répétitifs choisis parmi un motif répétitif représenté par la formule (9) suivante, un motif répétitif représenté par la formule (10) suivante et un motif répétitif représenté par la formule (11) suivante (dans lesquelles Q^{f1} représente une liaison simple, un atome d'oxygène, un groupe perfluoroalkylène en C₁₋₅ ou un groupe perfluoroalkylène en C₁₋₅ ayant un atome d'oxygène d'éther inséré entre des atomes de carbone-carbone, et, dans le cas où le polymère comprend essentiellement au moins deux types de motifs répétitifs, les Q^{f1} dans les formules peuvent être identiques ou différents, X² est un atome de fluor, ou un groupe représenté par -OR (où R est un atome d'hydrogène, un groupe alkyle en C₁₋₅, ou un groupe alkyle en C₁₋₅ ayant un atome d'oxygène d'éther inséré entre des atomes de carbone-carbone)) :

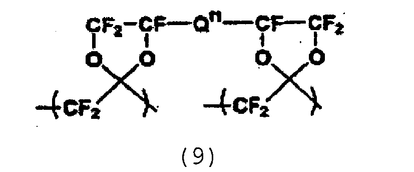

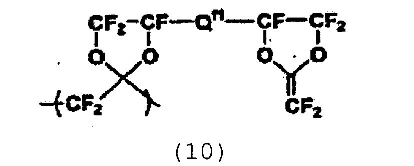

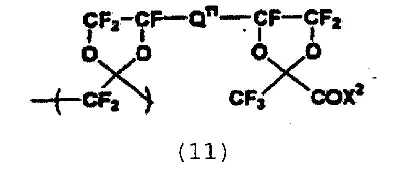

8. Procédé pour produire un polymère, **caractérisé par** la polymérisation d'un composé représenté par la formule (1A) suivante et/ou d'un composé représenté par la formule (1B) suivante, ou la polymérisation d'un composé représenté par la formule (1A) suivante et/ou d'un composé représenté par la formule (1B) suivante et d'un autre composé polymérisable (où Q^{f1} représente une liaison simple, un atome d'oxygène, un groupe perfluoroalkylène en C₁₋₅ ou un groupe perfluoroalkylène en C₁₋₅ ayant un atome d'oxygène d'éther inséré entre des atomes de carbone-carbone, et, dans le cas où le polymère comprend essentiellement au moins deux types de motifs répétitifs, les Q^{f1} dans les formules peuvent être identiques ou différents, X² est un atome de fluor, ou un groupe représenté par -OR (où R est un atome d'hydrogène, un groupe alkyle en C₁₋₅, ou un groupe alkyle en C₁₋₅ ayant un atome d'oxygène d'éther inséré entre des atomes de carbone-carbone)) :
